# EUROPEAN PATENT SPECIFICATION

(11) **EP 1 121 116 B1**
(45) Date of publication and mention of the grant of the patent: **02.01.2008**
(21) Application number: 99947489.3
(22) Date of filing: 12.10.1999
(51) Int. Cl.: A61K 31/22, A61K 9/20, A61K 31/366, A61P 3/06

(54) **SUSTAINED RELEASE PHARMACEUTICAL COMPOSITION OF FLUVASTATIN**
FLUVASTATIN-ARZNEIMITTEL MIT VERZÖGERTER WIRKSTOFFABGABE
COMPOSITION PHARMACEUTIQUE A LIBERATION DURABLE DE FLUVASTATINE

(30) Priority: 14.10.1998 US 172491
(43) Date of publication of application: 08.08.2001
(73) Proprietor: Novartis AG, 4056 Basel (CH); Novartis Pharma GmbH, 1230 Wien (AT)
(72) Inventor: SHAH, Rajen, Off Nagar Road, Pune 411014 (IN); PATEL, Arun, P., Succasunna, NJ 07876 (US); SANDRY, Roy, T., Hopatcong, NJ 07843 (US)
(74) Representative: de Weerd, Petrus G.W.
(86) International application number: PCT/EP1999/007627
(87) International publication number: WO 2000/021525

(56) References cited:
- EP-A- 0 465 096
- WO-A-97/23200
- WO-A-98/15264
- US-A- 4 915 954

## Description

### Field of the Invention

The present invention relates to sustained-release, oral dosage forms of pharmaceutical compositions.

### Background of the Invention

Conventional sustained-release, oral dosage forms of pharmaceutical compositions are utilized for a number of reasons. Such compositions provide for delivery of a pharmaceutically active agent over an extended time period, versus nonsustained-release, or immediate release, compositions, in which all of the pharmaceutically active agent is delivered over a short period of time immediately after the composition is ingested. Because this immediate release results in the active agent's peak concentration in the patient's system followed by concentrations reduced below therapeutically effective levels, nonsustained release compositions are typically administered in several, separate dosages throughout the day. Conventional sustained-release compositions therefore provide advantages over nonsustained-release compositions by providing the ability to reduce the number of doses required in a given period of time, e.g. single dosing versus multiple dosing, improving patient compliance, and providing a more constant active agent concentration in the blood over extended periods of time.

Although sustained release compositions may typically allow a single administration of the active agent's required dosage over a desired delivery period, for instance, a single daily dosage, such compositions may nonetheless exhibit premature release of significant amounts of the active agent. For a number of reasons, such a premature release, or "burst," of the pharmaceutically active agent can decrease the overall therapeutic efficiency of the active agent being delivered. One such problem occurs when the organ to which the active agent is delivered processes the active agent at a constant rate. Consequently, the premature release results in an amount of active agent in excess of the amount the organ is capable of processing in a given time, i.e. the organ is "flooded" with active agent. Much of the active agent may therefore pass by the organ without being processed and essentially is lost in the user's system where it provides no therapeutic affect.

U.S. Patent 5,376,383 teaches in Example 8 a matrix delivery system containing the therapeutic agent lovastatin, a hydroxypropyl cellulose (KLUCEL® LF), and a hydroxypropylmethyl cellulose (METHOCEL® E5 and METHOCEL® K15M). KLUCEL® LF, according to the manufacturer's literature, has a molecular weight range of about 95,000. At such a low molecular weight, the KLUCEL® LF is not known to have any effect on the matrix delivery system's release profile. The '383 patent remains silent regarding the release profile soon after administration.

It is desirable to develop a composition that provides all of the advantages of conventional sustained-release compositions, yet minimizes the premature release of significant amounts of active agent

### Brief Description of the Figures

Figure 1 is a graph depicting dissolution versus time of a composition in water, wherein the amount of hydroxypropylmethyl cellulose in the composition has been varied.
Figure 2 is a graph depicting dissolution versus time of compositions in acetate buffer, pH 4.0.
Figure 3 is a graph depicting dissolution versus time of compositions in phosphate buffer, pH 6.8.
Figure 4 is a graph depicting dissolution versus time of compositions in water.
Figure 5 is a graph depicting dissolution versus time of a composition containing both hydroxypropylmethyl cellulose and hydroxypropyl cellulose, wherein the dissolution medium is phosphate buffer, pH 6.8.
Figure 6 is a graph depicting dissolution versus time of a composition containing hydroxypropylmethyl cellulose but not hydroxypropyl cellulose, wherein the dissolution medium is phosphate buffer, pH 6.8.

### Summary of the invention

The present invention is directed to a pharmaceutical composition containing a pharmaceutically active agent, hydroxypropyl methyl cellulose, and a non-ionic, hydrophilic polymer selected from the group consisting of hydroxyethyl cellulose having a number average molecular weight ranging from 90,000 to 1,300,000, hydroxypropyl cellulose having a number average molecular weight of 370,000 to 1,500,000, and poly(ethylene- oxide) having a number average molecular weight ranging from 100,000 to 500,000.

The present invention is also directed to a method of releasing a pharmaceutically active agent in a mammal, wherein the method includes orally administering the pharmaceutically active agent to the mammal as part of a pharmaceutical composition including the pharmaceutically active agent, hydroxypropyl methyl cellulose; and a non-ionic, hydrophilic polymer selected from the group consisting of hydroxyethyl cellulose having a number average molecular weight ranging from 90,000 to 1,300,000, hydroxypropyl cellulose having a number average molecular weight of 370,000 to 1,500,000, and poly(ethylene oxide) having a number average molecular weight ranging from 370,000 to 500,000.

### Detailed Description of the Invention

It surprisingly has been discovered that compositions that contain, in addition to hydroxypropylmethyl cellulose (hereinafter "HPMC"), at least one non-ionic hydrophilic polymer, prevent premature release of the pharmaceutically active agent from the composition. As used herein, "premature release" means that a substantial amount of the pharmaceutically active agent is released in a short period of time after ingestion of the composition, for instance in a burst, such that the amount of active agent converted to a bioavailable form is in excess of the amount of the active agent that can be processed efficiently at the targeted active site. Prematurely released active agent may therefore bypass the targeted active site without being processed. As a result, therapeutic efficacy of the pharmaceutical composition may be reduced.

Non-ionic, hydrophilic polymers used in the pharmaceutical composition are selected from the group consisting of hydroxy ethylcellulose (hereinafter "HEC") having a number average molecular weight ranging from 90,000 to 1,300,000, preferably about 1,000,000 to about 1,300,000, hydroxypropylcellulose (hereinafter "HPC") having a number average molecular weight of 370,000 to 1,500,000, preferably 850,000 to 1,500,000, more preferably 1,000,000 to 1,200,000, and poly(ethylene oxide) (hereinafter "PEO") having a number average molecular weight ranging from 100,000 to 500,000, preferably 150,000 to 300,000, more preferably 200,000.

Examples ofHEC polymers are commercially available from Hercules Incorporated, Aqualon Division, under the tradename NATROSOL® 250H or NATROSOL® 250L. Examples of HPC polymers are also available from Hercules Incorporation, Aqualon Division under the tradename KLUCEL® or KLUCEL® HXF, and examples of PEO polymers are available from Union Carbide Corporation under the tradename POLYOX®. Methods of making the non-ionic, hydrophilic polymers suitable for use in the compositions described herein are known by those skilled in the art

The non-ionic, hydrophilic polymer may be present in the pharmaceutical composition in an amount ranging from about 1 to about 20 weight percent, preferably about 3 to about 12 weight percent, more preferably about 4 to about 7 weight percent. The non-ionic, hydrophilic polymer is present in an amount sufficient to prevent premature release of the pharmaceutically active agent.

As mentioned, the pharmaceutical compositions described herein also contain HPMC in an amount effective to provide sustained-release of the pharmaceutically active agent upon ingestion. As used herein, "sustained-release" means that the pharmaceutically active agent is released from the dosage form over an extended period of time, for example greater than about six hours. Preferably, the pharmaceutical compositions release less than about 80 weight percent of the active agent in the first eight hours after ingestion of the composition, with the balance of the pharmaceutically active agent being released thereafter. In preferred compositions, less than about 15 weight percent of the pharmaceutically active agent is released in the first 0.5 hour after ingestion, from about 10 to about 50 weight percent of the pharmaceutically active agent is released within about 2 hours after ingestion, and from about 40 to about 60 weight percent of the pharmaceutically active agent is released within about 6 hours after ingestion.

The pharmaceutical compositions comprise from about 15 to about 50 weight percent of HPMC, preferably from about 20 to about 40 weight percent of HPMC, based on total weight of the composition. The HPMC and the non-ionic, hydrophilic polymer preferably arc present at a weight ratio of HPMC to non-ionic, hydrophilic polymer ranging from about 10:1 to about 3:1, more preferably from about 7:1 to about 5: 1, and even more preferably about 6:1.

One HPMC polymer useful in the pharmaceutical composition described herein is available commercially from Dow Chemical under the trade name METHOCEL®. Preferably, the HPMC will have a hydroxypropyl (HP) degree of substitution up to about 12, i.e., the HPMC will comprise up to about 12 percent HP functionality. Preferably, the HPMC will comprise from about 7 to about 12 percent HP functionality, and more preferably from about 7 to about 9 percent HP- The HPMC preferably will have normal viscosity (2.0% HPMC in water) of from about 100 to about 100,000 cps and a number average molecular weight of about 20,000 to about 170,000. A particularly preferred HPMC is METHOCEL® K100LV, which has a number average molecular weight of about 20,000 to about 30,000. Methods of making such HPMC polymers are well known by those skilled in the art.

Upon ingestion, the non-ionic, hydrophilic polymer and the HPMC form a gel matrix in which the active agent is contained. The pharmaceutically active agent is then released from the gel matrix over time, thereby providing sustained-release of the active agent, such that a substantial amount of the released active agent may be processed efficiently at the targeted active site. Preferably, the gel matrix has sufficient strength to prevent substantial premature degradation of the matrix. The get matrix should also be formed within a time period that is effective to prevent the premature release of the active agent prior to formation of the gel matrix. For example, the gel matrix preferably forms within about 5 minutes after ingestion of the composition to prevent a burst of active agent prior to gel formation. It is believed that the nonionic, hydrophilic polymer operates to decrease the rate of gel formation to an acceptable level.

Typical pharmaceutically active agents which may be administered via the instant invention include, but are not limited to: (a) central nervous system (CNS) agents, such as antipsychotics, anticonvulsants, including carbamazepine and oxcarbazepine, antidepressants, antiepileptics, anxiolytics, and hypnotics; (b) cardiovascular agents, such as anti-arrhythmics, hypolipedermics, anti-anginals, anti-coagulants, anti-hypertensives, antiplatelets, diuretics, and electrolytes (Ca, K, Mg); and (c) antiinflammatories, antiasthmatics, antiarthritics, oral hypoglycemics, and aromatase inhibitors; to name a few.

The pharmaceutically active agents that can be delivered include inorganic and organic compounds without limitation, including drugs that act on the peripheral nerves, adrenergic receptors, cholinergic receptors, nervous system, skeletal muscles, cardiovascular, smooth muscles, blood circulatory system, synaptic sites, neuroeffector junctional sites, endocrine and hormone systems, immunological system, reproductive system, skeletal system, alimentary and excretory systems, inhibitory of hormonal and histamine systems, those materials that act on the central nervous system, such as antidepressants, including amiflamine, amitriptyline, alaproclate, protriptyline, doxepin, imiprimine, trazodine, paprotiline, zimelidine, fluvoxamine; antipsychotic-neuroleptic agents such as chlorpromazine, haloperidol, thioridazine, trifluoperazine, MK-0212, remoxipride, anticonvulsants, such as carbamazepine, oxcarbamazepine, phenytoin, phenobarbital; sedative-hypnotic agents, such as triazolam, chlordiazepoxide, temazepam, chlorazepate, alprazolam, diazepam, flurazepam, lorazepam, oxazepam, hydroxyzine, prazepam, meprobamate, butalbital, orphenadrine, chlorzoxazone, cyclobenzaprine; antiparkinson agents, such as benztropine, carbidopa, levodopa, L 647-339; analgesics, such as acetaminophen, oxycodone, Hydrocodone, codeine, and propoxyphen. Respiratory agents, including sympathomimetics, brochodilators, antihistamines; and antiasthmatics, such as diethylpropion, ephedrine, epinephrine, isoproterenol, metaproterenol, terbutaline, cyproheptadine, azatadine, diphenhydramine, promethazine, chlorpheniramine, brompheniramine, aminophylline, theophylline, albuterol, tranilast, enprefylline, and budesonide, also may be used. Cardiovascular and antihypertensive agents, including coronary vasodilators, cardiac glycosides, betablockers, slow calcium channel blockers, antiarrhythmics, peripheral vasodilators such as isosorbide dinitrate, nitroglycerin, dipyridamole, digoxin, nadolol propranolol, metaprolol, atenolol, timolol, disopyramide, procainamide, nifedipine, quinidine, lidocaine, diltiazam, verapamil, prazosin, clinidine, hydralazine, methyldopa, captopril, metyresine, enalapril, lysinopril, felodipine, tocainide, also may be used- Diuretics, such as amiloride, spiranolactone, hydrochlorothiazide, chlorothiazide, acetazolamide, chlorthalidone, metolazone, furosemide, triamterene, methyclothiazide, ethacrynic acid, indacrinone; antiartereosclerotic agents, such as conjugated estrogens, estradiol, ethinyl estradiol, diethylstilbesterol; progestins, such as progesterone, hydroxyprogesterone, medroxyprogesterone, norethindrone; glucocorticoids and mineralocorticoids, such as hydrocortisone, betamethasone, dexamethasone, methylprednisolone, prednisolone, prednisone, triamcinolone, and MK-0621, also may be used. Nonsteroidal anti-inflammatory agents, antiarthritic and antigout agents, such as allopurinol, aspirin, fenprofen, ibuprofen, indomethacin, naproxen, phenylbutazone, sulindac, tolmetin, diflunisol, piroxicam, meclofenamate, penicillamine, probenecid, and colchicine; gastrointestinal agents, including anticholinergics, antispasmodics, antidiarrheal; and antiulcer histamine-H₂-antagonists, such as bethanechol, clidinium, dicyclomine, meclizine, prochlorperazine, trimethobenzamide, loperamide, cimetadine, ranitidine, diphenoxylate, famotidine, and omeprazole; oral hypoglycemics, such as chlorpropamide tolazamide and tolbutamide; anticoagulants, such as warfarin, phenindione, and anisindione; anti-infective agents, including antibiotic, antimicrobial, antiviral, antiparasitic; and antifungal agents, such as cefoxitin, thiabendazole, cephalexin, tetracycline, ampicillin, amoxicillin, sulfamethoxacole, cefaclor, erythromycin, penicillin, nitrofurantoin, minocycline, doxycycline, cefadroxil miconazole, phenazopyridine, norfloxacin, clorsulon, fludalanine, pentizidone, cilastin, phosphonomycin, ivermectin, imipenem, arprinocid, and foscamet; nutritional supplements, including vitamins such as isotretinion (Vit. A), Vit. D, tocopherols (Vit. E), and phytonadione (Vit. K); amino acids, such as L-tryptophan and L-lysine; and lipids, such as com oil and medium chain triglycerides, also may be used. Another class of pharmaceutical agents which may be used include those agents which aid in the reduction of cholesterol in humans.

The pharmaceutically active agents previously listed may be present in the pharmaceutical composition in an amount ranging from about 0.1 to about 80 weight percent, preferably about 10 to about 50 weight percent, more preferably about 20 to about 40 weight percent.

A class of pharmaceutically active agents known as HMG-CoA reductase inhibitors are known for use in certain pharmaceutical compositions to enhance the lowering of plasma cholesterol level in humans. Methods of making the HMG-CoA reductase inhibitors are well known by those skilled in the art and such agents include those commercially available as fluvastatin (available from Novartis Pharmaceuticals, Inc. under the trade name LESCOL®), simvastatin (available from Merck & Co., Inc. under the trade name ZOCOR®), atorvastatin (available from Warner-Lambert under the trade name LIPITOR®), pravastatin (available from Bristol-Myer Squibb under the trade name PRAVACHOL®), cerivastatin (available from BASF under the trade name LIPOBAY®), lovastatin (available from Merck & Co., Inc. under the trade name MEVACOR®) and mevastatin. The HMG-CoA reductase inhibitors may be used in their free acid forms, in their ester forms, or as their pharmaceutically acceptable salts. Such pharmaceutically acceptable salts include, for example, sodium salts, calcium salts, and ester salts.

The HMG-CoA reductase inhibitors may be used as racemic mixtures, or as a more active stereoisomer as appropriate. For example, a racemic mixture of 3-*R*-5-*S*-fluvastatin sodium and 3-*S*-5-*R* fluvastatin sodium may be used, although the stereoisomer 3-*R*-5-*S-*fluvastatin sodium has been found to be the more active form.

The HMG-CoA reductase inhibitor may be present in an amount effective to inhibit biosynthesis of cholesterol in humans. In one embodiment, the pharmaceutical compositions comprise from about 5 to about 50 weight percent of the HMG-CoA reductase inhibitor, based on total weight of the composition. More preferably, the compositions comprise from about 20 to about 40 weight percent of the HMG-CoA reductase inhibitors, based on total weight of the composition.

Other ingredients which may be incorporated into the compositions to facilitate processing and/or provide enhanced properties of the composition include well-known tableting binders (e.g, gelatin, sugars, natural and synthetic gums, polyvinylpyrrolidone), disintegrants (e.g., croscarmelose, crospovidone, sodium starch glycolate), lubricants (e.g., magnesium stearate, hydrogenated vegetable oil, carnauba wax) ; flow agents (e.g., silicon dioxide), anti-adherents or glidants (e.g., talc) as well as sweeteners, coloring mediums (e.g., iron oxide, aluminum flakes), filler materials (e.g., lactose and other carbohydrates, pregelitinized starch, potassium bicarbonate), flavoring mediums, and antioxidants. Selection of a particular ingredient or combinations of ingredients and the amounts used wilt be readily determinable by one skilled in the art by reference to standard procedures and practices for preparing tableted or encapsulated or other dosage forms.

The pharmaceutical compositions described herein may be administered to mammals, more particularly humans, as treatments associated with the particular pharmaceutically active agents included therein.

### Example 1

A portion of fluvastatin sodium is calculated and weighed. Potassium bicarbonate, microcrystalline cellulose, povidone, HPC, and HPMC are weighed and placed into individual separately labeled containers. A 20 weight percent excess of the batch quantity of OPADRY® Yellow, YS-1-6347-G, is then placed into a labeled container. The microcrystalline cellulose, fluvastatin sodium, povidone, HPC, and HPMC are transferred, in that order, into a collette gral and mixed for 5 minutes with the plow at slow speed and the chopper off. The resulting mixture is passed through a 0-033 inch screen using a tornado mill with knives forward and at a slow speed. The screened material is then mixed again in a collette gral with the plow at slow speed and the chopper off.

Potassium bicarbonate is dissolved into purified water until a clear homogenous solution is obtained. The potassium bicarbonate solution is then combined with the screened material, and the resulting mixture is granulated in a collette gral with the plow at fast speed and the chopper at slow speed. After adding the above solution, granulation should continue for 30 seconds with the plow at fast speed and the chopper at slow speed and for another 30 seconds with the plow at fast speed and the chopper at fast speed. The granulated mixture is then dried in a fluid bed dryer using a target inlet temperature of 50 degrees C until an LOD of 2 percent to 3 percent is obtained.

The dried granules are then passed through a 1/16 inch screen using a tornado mill with knives forward and at slow speed. An amount of magnesium stearate based on the proportion of actual yield from the 1/16 inch screening step to the theoretical yield from the same step is calculated and weighed. The weighed magnesium stearate is then passed through a 60 mesh screen and blended with the dried granules in a free fall blender and the resulting granulation blend discharged into a plastic lined labeled drum. The granulation blend is then compressed into tablets and the tablets are dedusted, passed through a metal checker, and stored in a plastic labeled drum.

To coat the tablets, the OPADRY® Yellow is mixed with a required quantity of purified water to obtain a 10 w/w percent suspension. The tablets are transferred to a coating pan and warmed to a temperature of 40-45 degrees C. The OPADRY® Yellow suspension is then added, to spray coat the tablets until a 3 percent solid weight gain per tablet is achieved. The coating spray is shut off, and the tablets are cooled by shutting off the pan heat and jogging the pan for 5 minutes.

### Example 2

84.24 mg of fluvastatin sodium were combined with the following excipients according to the method described in Example I to provide a single dosage form described in Table 1:

**TABLE 1 -**

| | |
|---|---|
| Fluvastatin sodium | 84.24 mg |
| Potassium bicarbonate, USP | 8-42 mg |
| Microcrystalline cellulose, NF, PH101 (AVICEL®) | 111.26 mg |
| Povidone, USP | 4.88 mg |
| HPC,NF (KLUCEL® HXF) | 16-25 mg |
| HPMC, USP (METHOCEL® K 100LV) | 97-50 mg |
| Magnesium Stearate | 2.44 mg |
| OPADRY® Yellow | 9.75 mg |

### Example 3

84.25 mg of fluvastatin sodium were combined with the following excipients by the method described in Example 1 to provide a single dosage form described in Table 2:

**TABLE 2**

| | |
|---|---|
| Fluvastatin sodium | 84.25 mg |
| Potassium bicarbonate, USP | 8.42 mg |
| Microcrystalline cellulose, NF, PH101 (AVICEL®) | 1112 mg |
| Povidone, USP | 4.88 mg |
| HPC, HF (KLUCEL® HXF) | 16.25 mg |
| HPMC, USP (METHOCEL® K 100LV) | 32.50 mg |
| HPMC, USP (METHOCEL® K15M) | 3250 mg |
| HPMC, USP (METHOCEL® K4M) | 32.50 mg |
| Magnesium Stearate, NF | 2.44 mg |
| OPADRY® Yellow, YS-1-6347-G | 9.75 mg |

### Example 4

168.48 mg of fluvastatin sodium were combined with the following excipients according to the method described in Example 1 to provide a single dosage form described in Table 3:

**TABLE 3**

| | |
|---|---|
| Fluvastatin sodium | 168-48 mg |
| Potassium bicarbonate, USP | 8.42 mg |
| Microcrystalline cellulose, NF, PH101 (AVICEL®) | 65 mg |
| Povidone, USP | 20.5 mg |
| HPC, NF (KLUCEL® HXF) | 20.5 mg |
| HPMC, USP (METHOCEL® K 100LV) | 110.7 mg |
| HPMC, USP (METHOCEL®) | 123 mg |
| Magnesium stearate, NF (1%) | 4.1 mg |
| OPADRY® Red | 123 mg |

### Example 5

Dosage forms of the pharmaceutical composition described in Example 2 were prepared, while varying the weight percentage of HPMC from 30 weight percent to 10 weight percent in 5 weight percent increments. Each dosage form was then tested for its dissolution in water while stirring at a paddle speed of 50 rpm, at a temperature of 37°C.

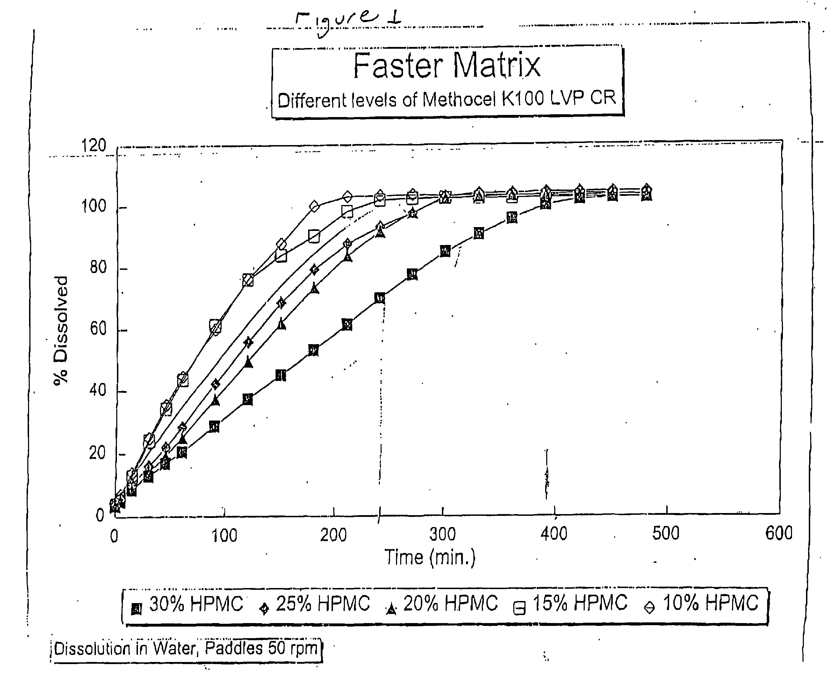

The results of each experiment were plotted in a graph as percentage dissolution versus time as shown in Figure 1.

### Comparative Example 1

A dosage form having the composition described below in Table 4 was prepared according to the method described in Example 1:

**TABLE 4**

| | |
|---|---|
| Fluvastatin sodium | 42.12 mg |
| Sodium bicarbonate | 4.21 mg |
| Microcrystalline cellulose, NF (PH01) | 146-17 mg |
| Povidone | 625 mg |
| HPC, NF (KLUCEL® HXF) | 50.00 mg |
| Magnesium stearate NF | 1.25 mg |
| OPADRY® Yellow | 10.00 mg |

### Comparative Example 2

A dosage form having the composition shown below in Table 5 was prepared according to the method described in Example 1:

**TABLE 5**

| | |
|---|---|
| Fluvastatin sodium | 42.12 mg |
| Sodium bicarbonate | 4.21 mg |
| Microcrystalline cellulose, NF (PH01) | 118.67 mg |
| Povidone | 6.25 mg |
| HPMC, NF (METHOCEL® HXF) | 7750 mg |
| Magnesium stearate NF | 1.25 mg |
| OPADRY® Yellow | 10.00 mg |

### Comparative Example 3

The dosage forms of Example 2, Comparative Example 1, and Comparative Example 2 were tested for their dissolution at a temperature of 37°C by placing each dosage form in 100 mM acetate buffer and stirring at a paddle speed of 50 rpm.

The acetate buffer contained 4.0 grams of sodium hydroxide dissolved in about 450 milliliters of water. The pH was adjusted to 4.0 by the addition of acetic acid, and the solution was diluted to one liter with distilled water.

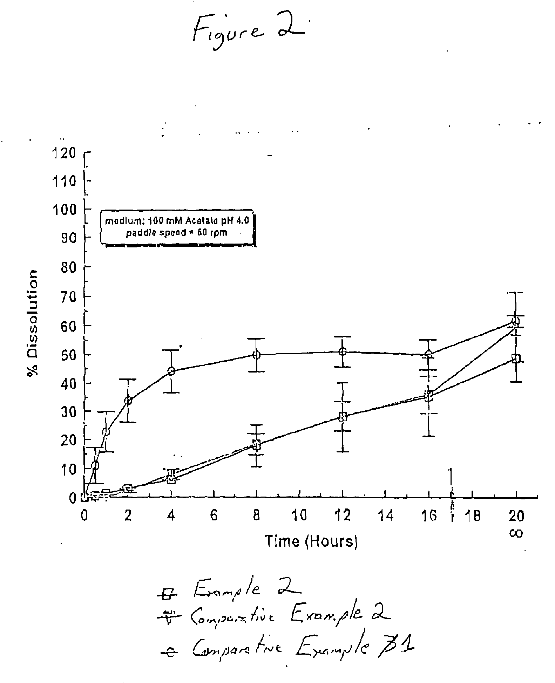

The dissolution data were plotted in a graph as percentage dissolution versus time as shown in Figure 2. As can be seen from the plot, the fluvastatin composition of Comparative Example 1 containing HPC but no HPMC showed an undesirably high rate of dissolution as compared to the composition of Example 2.

### Comparative Example 4

The dosage forms of Example 2, Comparative Example 1, and Comparative Example 2 were tested for their dissolution at a temperature of 37°C by placing each dosage form in 50 mM phosphate buffer, pH 6.8, and stirring at paddle speeds of 50 rpm and 100 rpm.

The phosphate buffer contained 3.312 grams of monobasic sodium phosphate monohydrate and 3.692 grams of dibasic sodium phosphate anhydrous dissolved in about 500 milliliters of water. The resulting solution was diluted to one liter with distilled water.

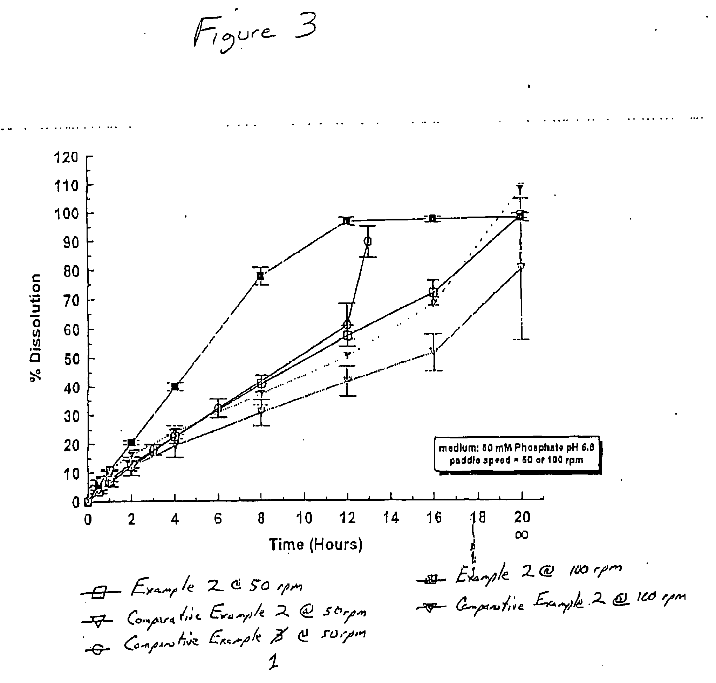

The dissolution data were plotted in a graph as percentage dissolution versus time as shown in Figure 3. As can be seen from the plot, the fluvastatin composition of Example 2 showed a release profile comparable at a stirring speed of 50 rpm to the fluvastatin compositions having only one of IIPMC or HPC.

### Comparative Example 5

The dosage forms of Example 2, Comparative Example 1, and Comparative Example 2 were tested for their dissolution at a temperature of 37°C by placing each dosage form distilled water and stirring at a paddle speed of 50 rpm.

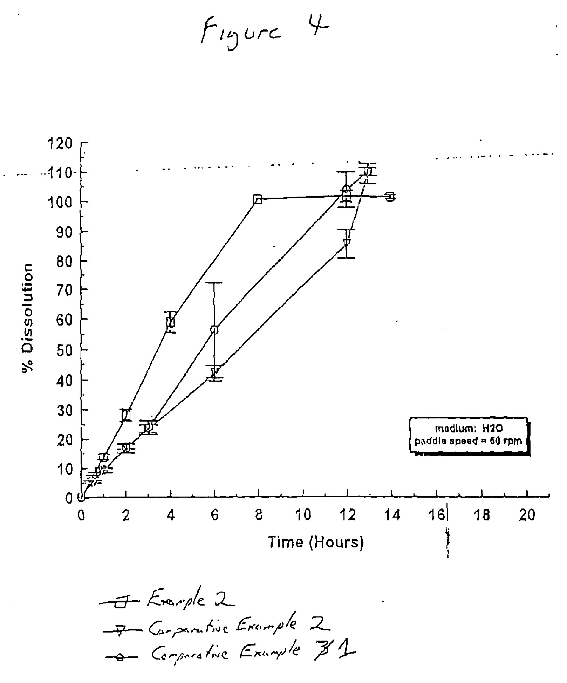

The results or each experiment were plotted in a graph as percentage dissolution versus time as shown in Figure 4. As can be seen from the plot, the fluvastatin composition of Example 2 showed a dissolution profile comparable to the fluvastatin compositions having only one of HPMC or HPC.

### Comparative Example 6

The dosage forms of Example 2 and Comparative Example 2 were repeatedly tested for their dissolution at a temperature of 37°C by placing each dosage form in 50 mM phosphate buffer, pH 6.8, and stirring at a paddle speed of 50 rpm.

The phosphate buffer contained 3312 grams of monobasic sodium phosphate monohydrate and 3.692 grams of dibasic sodium phosphate anhydrous dissolved in about 500 milliliters of water. The resulting solution was diluted to one liter with distilled water.

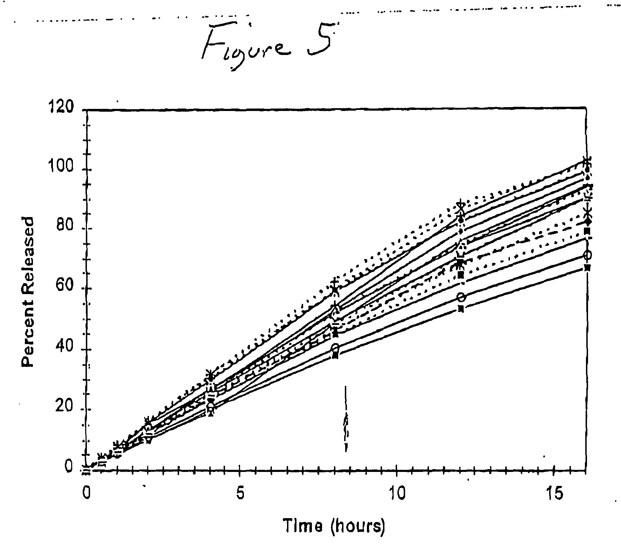

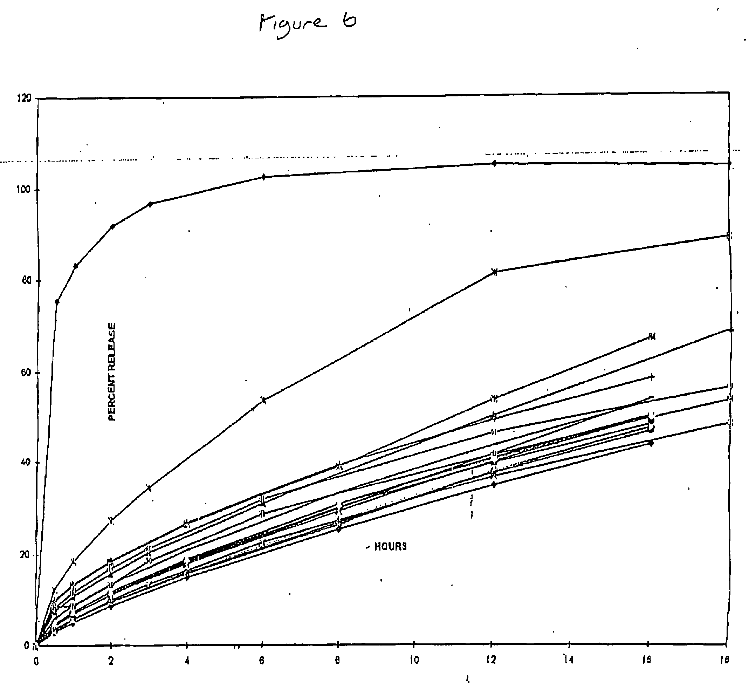

Dissolution data for Example 2 and Comparative Example 2 were plotted on a graph as percentage dissolution versus time as shown in Figures 5 and 6, respectively. A comparison of Figures 5 and 6 shows that the composition of Example 2, containing both HPMC and HPC, showed better reproducibility in its dissolution profile than the composition of Comparative Example 2, which contained only HPMC.

## Claims

1. A pharmaceutical composition, comprising:
fluvastatin or a pharmaceutically acceptable salt thereof hydroxypropyl methyl cellulose; and
a non-ionic, hydrophilic polymer selected from the group consisting of hydroxyethyl cellulose having a number average molecular weight ranging from 90,000 to 1,300,000, hydroxypropyl cellulose having a number average molecular weight of 370,000 to 1,500,000, and poly(ethylene oxide) having a number average molecular weight ranging from 100,000 to 500,000.

2. A pharmaceutical composition of claim 1, wherein the non-ionic, hydrophilic polymer is selected from the group consisting of hydroxyethyl cellulose having a number average molecular weight ranging from 1,000,000 to 1,300,000, hydroxypropyl cellulose having a number average molecular weight of 850,000 to 1,500,000, and poly(ethylene oxide) having a number average molecular weight ranging from 150,000 to 300,000.

3. A pharmaceutical composition according to claim 2 wherein the hydroxypropyl cellulose has a number average molecular weight ranging from 1,000,000 to 1,200,000.

4. A pharmaceutical composition of claim 1, wherein the non-ionic, hydrophilic polymer is hydroxypropyl cellulose having a number average molecular weight of about 1,150,000.

5. A pharmaceutical composition according to claim 2 wherein the poly(ethylene oxide) has a number average molecular weight of 200,000.

6. A pharmaceutical composition of claim 1, comprising from about 5 to about 50 weight percent of fluvastatin or a pharmaceutically acceptable salt thereof, based on total weight of said composition.

7. A pharmaceutical composition of claim 1, comprising from about 20 to about 40 weight percent of fluvastatin or a pharmaceutically acceptable salt thereof, based on total weight of said composition.

8. A pharmaceutical composition of claim 1, comprising from about 15 to about 50 weight percent of said hydroxypropyl methyl cellulose, based on total weight of said composition.

9. A pharmaceutical composition of claim 1, comprising from about 20 to about 40 weight percent of said hydroxypropyl methyl cellulose, based on total weight of said composition.

10. A pharmaceutical composition of claim 1, comprising from about 3 to about 12 weight percent of said non-ionic hydrophilic polymer, based on total weight of said composition.

11. A pharmaceutical composition of claim 1, comprising from about 4 to about 7 weight percent of said non-ionic hydrophilic polymer, based on total weight of said composition.

12. A pharmaceutical composition of claim 1, wherein the weight ratio of said hydroxypropyl methyl cellulose to said non-ionic hydrophilic polymer ranges from about 10:1 to about 3:1.

13. A pharmaceutical composition of claim 1, wherein the weight ratio of said hydroxypropyl methyl cellulose to said non-ionic hydrophilic polymer is about 7:1 to about-5:1.

14. A pharmaceutical composition of claim 1 wherein the weight ratio of said hydroxypropyl methyl cellulose to said non-ionic hydrophilic polymer is 6/1.

15. Pharmaceutical composition according to any of the previous claims, comprising;
84.24 mg of Fluvastatin sodium,
8.42 mg Potassium bicarbonate,
111.26 mg Microcrystalline Cellulose, NF, PH101, AVICEL®,
4.88 mg Povidone,
16.25 mg HPC, NF, KLUCEL© HXF,
97.50 mg HPMC, METHOCEL® K 100LV,
2.44 mg Magnesium stearate, and
9.75 mg OPADRY ® Yellow.

16. Pharmaceutical composition according to any of claims 1 to 14, comprising:
84.25 mg Fluvastatin sodium
8.42 mg Potassium bicarbonate,
111,2 mg Microcrystalline cellulose, NF, PH101, AVICEL®,
4.88 mg Povidone,
16.25 mg HPC, HF, KLUCEL® HXF,
32.50 mg HPMC, METHOCEL® K 100LV,
32.50 mg HPMC, METHOCEL® K 15M,
32.50 mg HPMC, METHOCEL® K 4M,
2.44 mg Magnesium stearate, NF, and
9.75 mg OPADRY® Yellow, YS-1-6347-G.

17. Pharmaceutical composition according to any of claims 1 to 14, comprising:
168.48 mg Fluvastatin sodium,
8.42 mg Potassium bicarbonate,
65 mg Microcrystalline cellulose, NF, PH101, AVICEL®,
20.5 mg Povidone,
20.5 mg HPC, NF, KLUCEL® HXF,
110.7 mg HPMC, METHOCEL® K 100LV,
12.3 mg HPMC, METHOCEL®,
4-1 mg Magnesium stearate, NF (1 %), and
12.3 mg OPADRY® Red.

18. Use of a composition, comprising:
fluvastatin or a pharmaceutically acceptable salt thereof;
hydroxypropyl methyl cellulose; and
a non-ionic, hydrophilic polymer selected from the group consisting of hydroxyethyl cellulose having a number average molecular weight ranging from 90,000 to 1,300,000, hydroxypropyl cellulose having a number average molecular weight ranging from 370,000 to 1,500,000, and poly(ethylene oxide) having a number average molecular weight ranging from 100,000 to 500,000 for the manufacture of a medicament for the reduction of plasma cholesterol levels.

19. Use according to claim 18, wherein the non-ionic, hydrophilic polymer is selected from the group consisting of hydroxyethyl cellulose having a number average molecular weight ranging from 1,000,000 to 1,300,000, hydrox-ypropyl cellulose having a number average molecular weight ranging from 850,000 to 1,500,000, and poly(ethylene oxide) having a number average molecular weight ranging from 150,000 to 300,000.

20. Use according to claim 19 wherein the hydroxypropyl cellulose has a number average molecular weight ranging from 1,000,000 to 1,200,000.

21. Use according to claim 19, wherein the non-ionic, hydrophilic polymer is hydroxypropyl cellulose with a number average molecular weight of about 1,150,000.

22. Use according to claim 19 wherein the poly(ethylene oxide) has a number average molecular weight of about 200,000.

23. Use according to any of claims 18 to 21, wherein the composition comprises, based on the total weight of said composition, from about 5 to about 50 per cent of fluvastatin or a pharmaceutically acceptable salt thereof.

24. Use according to claim 23, wherein the composition comprises, based on the total weight of said composition, from about 20 to about 40 per cent of fluvastatin or a pharmaceutically acceptable salt thereof.

25. Use according to any of claims 18 to 24, wherein the composition comprises, based on the total weight of said composition, from about 15 to about 50 per cent of said hydroxypropyl methyl cellulose.

26. Use according to claim 25, wherein the composition comprises, based on the total weight of said composition, from about 20 to about 40 per cent of said hydroxypropyl methyl cellulose.

27. Use according to any of claims 18 to 26, wherein the pharmaceutical composition comprises, based on the total weight of said composition, from about 3 to about 12 per cent of said non-ionic, hydrophilic polymer.

28. Use according to claim 27, wherein the pharmaceutical composition comprises, based on the total weight of said composition, from about 4 to about 7 per cent of said non-ionic, hydrophilic polymer.

29. Use according to claim 18, wherein the weight ratio of said hydroxypropyl methyl cellulose to said non-ionic hydrophilic polymer ranges from about 10:1 to about 3:1.

30. Use according to claim 29, wherein the weight ratio of said hydroxypropyl methyl cellulose to said non-ionic hydrophilic polymer ranges from about 7:1 to about 5:1.

31. Use according to claim 29 wherein the weight ratio of said hydroxypropyl methyl cellulose to said non-ionic hydrophilic polymer is 6/1.

32. A pharmaceutical composition according to claim 1 wherein the HPMC has a hydroxypropyl (HP) degree of substitution from about 7 to 12%.

33. A pharmaceutical composition according to claim 1 wherein the HPMC has a hydroxypropyl (HP) degree of substitution from about 7 to 9 percent HP.

## Patentansprüche

1. Pharmazeutische Zusammensetzung, umfassend
Fluvastatin oder ein pharmazeutisch akzeptables Salz hiervon, Hydroxypropylmethylcellulose, und
ein nicht ionisches hydrophiles Polymer, das ausgewählt ist aus der Gruppe, die besteht aus Hydroxyethylcellulose mit einem zahlenmittleren Molekulargewicht im Bereich von 90.000 bis 1.300.000, Hydroxypropylcellulose mit einem zahlenmittleren Molekulargewicht von 370.000 bis 1.500.000 und Poly-(ethylenoxid) mit einem zahlenmittleren Molekulargewicht im Bereich von 100.000 bis 500.000.

2. Pharmazeutische Zusammensetzung nach Anspruch 1, worin das nicht ionische hydrophile Polymer ausgewählt ist aus der Gruppe, die besteht aus Hydroxyethylcellulose mit einem zahlenmittleren Molekulargewicht im Bereich von 1.000.000 bis 1.300.000, Hydroxypropylcellulose mit einem zahlenmittleren Molekulargewicht von 850.000 bis 1.500.000 und Poly(ethylenoxid) mit einem zahlenmittleren Molekulargewicht im Bereich von 150.000 bis 300.000.

3. Pharmazeutische Zusammensetzung nach Anspruch 2, worin die Hydroxypropylcellulose ein zahlenmittleres Molekulargewicht im Bereich von 1.000.000 bis 1.200.000 hat.

4. Pharmazeutische Zusammensetzung nach Anspruch 1, worin das nicht ionische hydrophile Polymer Hydroxypropylcellulose mit einem zahlenmittleren Molekulargewicht von etwa 1.150.000 ist.

5. Pharmazeutische Zusammensetzung nach Anspruch 2, worin das Poly(ethylenoxid) ein zahlenmittleres Molekulargewicht von 200.000 hat.

6. Pharmazeutische Zusammensetzung nach Anspruch 1, umfassend etwa 5 bis etwa 50 Gew.-% Fluvastatin oder ein pharmazeutisch akzeptables Salz hiervon, bezogen auf das Gesamtgewicht dieser Zusammensetzung.

7. Pharmazeutische Zusammensetzung nach Anspruch 1, umfassend etwa 20 bis etwa 40 Gew.-% Fluvastatin oder ein pharmazeutisch akzeptables Salz hiervon, bezogen auf das Gesamtgewicht dieser Zusammensetzung.

8. Pharmazeutische Zusammensetzung nach Anspruch 1, umfassend etwa 15 bis etwa 50 Gew.-% der Hydroxypropylmethylcellulose, bezogen auf das Gesamtgewicht dieser Zusammensetzung.

9. Pharmazeutische Zusammensetzung nach Anspruch 1, umfassend etwa 20 bis etwa 40 Gew.-% der Hydroxypropylmethylcellulose, bezogen auf das Gesamtgewicht dieser Zusammensetzung.

10. Pharmazeutische Zusammensetzung nach Anspruch 1, umfassend etwa 3 bis etwa 12 Gew.-% des nicht ionischen hydrophilen Polymers, bezogen auf das Gesamtgewicht dieser Zusammensetzung.

11. Pharmazeutische Zusammensetzung nach Anspruch 1, umfassend etwa 4 bis etwa 7 Gew.-% des nicht ionischen hydrophilen Polymers, bezogen auf das Gesamtgewicht dieser Zusammensetzung.

12. Pharmazeutische Zusammensetzung nach Anspruch 1, worin das Gewichtsverhältnis von Hydroxypropylmethylcellulose zu nicht ionischem hydrophilem Polymer von etwa 10:1 bis etwa 3:1 reicht.

13. Pharmazeutische Zusammensetzung nach Anspruch 1, worin das Gewichtsverhältnis von Hydroxypropylmethylcellulose zu nicht ionischem hydrophilem Polymer von etwa 7:1 bis etwa 5:1 reicht.

14. Pharmazeutische Zusammensetzung nach Anspruch 1, worin das Gewichtsverhältnis von Hydroxypropylmethylcellulose zu nicht ionischem hydrophilem Polymer 6:1 beträgt.

15. Pharmazeutische Zusammensetzung nach einem der vorhergehenden Ansprüche, umfassend 84,24 mg Fluvastatinnatrium,
8,42 mg Kaliumbicarbonat,
111,26 mg mikrokristalline Cellulose, NF, PH1 01, Avicel^{®},
4,88 mg Povidon,
16,25 mg HPC, NF, Klucel^{®} HXF,
97,50 mg HPMC, Methocel^{®} K 100LV,
2,44 mg Magnesiumstearat und
9,75 mg Opadry^{®} Gelb.

16. Pharmazeutische Zusammensetzung nach einem der Ansprüche 1 bis 14, umfassend
84,25 mg Fluvastatinnatrium,
8,42 mg Kaliumbicarbonat,
111,2 mg mikrokristalline Cellulose, NF, PH101, Avicel^{®},
4,88 mg Povidon,
16,25 mg HPC, HF, Klucel^{®} HXF,
32,50 mg HPMC, Methocel^{®} K 100LV,
32,50 mg HPMC, Methocel^{®} K 15M,
32,50 mg HPMC, Methocel^{®} K 4M,
2,44 mg Magnesiumstearat, NF, und
9,75 mg Opadry^{®} Gelb, YS-1-6347-G.

17. Pharmazeutische Zusammensetzung nach einem der Ansprüche 1 bis 14, umfassend
168,48 mg Fluvastatinnatrium,
8,42 mg Kaliumbicarbonat,
65 mg mikrokristalline Cellulose, NF, PH101, Avicel^{®},
20,5 mg Povidon,
20,5 mg HPC, NF, Klucel^{®} HXF,
110,7 mg HPMC, Methocel^{®} K 100LV,
12,3 mg HPMC, Methocel^{®},
4,1 mg Magnesiumstearat, NF (1 %) und
12,3 mg Opadry^{®} Rot.

18. Verwendung einer Zusammensetzung, umfassend
Fluvastatin oder ein pharmazeutisch akzeptables Salz hiervon, Hydroxypropylmethylcellulose, und
ein nicht ionisches hydrophiles Polymer, das ausgewählt ist aus der Gruppe, die besteht aus Hydroxyethylcellulose mit einem zahlenmittleren Molekulargewicht im Bereich von 90.000 bis 1.300.000, Hydroxypropylcellulose mit einem zahlenmittleren Molekulargewicht von 370.000 bis 1.500.000 und Poly(ethylenoxid) mit einem zahlenmittleren Molekulargewicht im Bereich von 100.000 bis 500.000, zur Herstellung eines Arzneimittels für die Reduktion von Plasmacholesterinspiegeln.

19. Verwendung nach Anspruch 18, worin das nicht ionische hydrophile Polymer ausgewählt ist aus der Gruppe, die besteht aus Hydroxyethylcellulose mit einem zahlenmittleren Molekulargewicht im Bereich von 1.000.000 bis 1.300.000, Hydroxypropylcellulose mit einem zahlenmittleren Molekulargewicht im Bereich von 850.000 bis 1.500.000 und Poly(ethylenoxid) mit einem zahlenmittleren Molekulargewicht im Bereich von 150.000 bis 300.000.

20. Verwendung nach Anspruch 19, worin die Hydroxypropylcellulose ein zahlenmittleres Molekulargewicht im Bereich von 1.000.000 bis 1.200.000 hat.

21. Verwendung nach Anspruch 19, worin das nicht ionische hydrophile Polymer Hydroxypropylcellulose mit einem zahlenmittleren Molekulargewicht von etwa 1.150.000 ist.

22. Verwendung nach Anspruch 19, worin das Poly(ethylenoxid) ein zahlenmittleres Molekulargewicht von etwa 200.000 hat.

23. Verwendung nach einem der Ansprüche 18 bis 21, worin die Zusammensetzung etwa 5 bis etwa 50 % Fluvastatin oder pharmazeutisch akzeptables Salz hiervon umfasst, bezogen auf das Gesamtgewicht dieser Zusammensetzung.

24. Verwendung nach Anspruch 23, worin die Zusammensetzung etwa 20 bis etwa 40 % Fluvastatin oder ein pharmazeutisch akzeptables Salz hiervon umfasst, bezogen auf das Gesamtgewicht dieser Zusammensetzung.

25. Verwendung nach einem der Ansprüche 18 bis 24, worin die Zusammensetzung etwa 15 bis etwa 50 % der Hydroxypropylmethylcellulose umfasst, bezogen auf das Gesamtgewicht dieser Zusammensetzung.

26. Verwendung nach Anspruch 25, worin die Zusammensetzung etwa 20 bis 40 % der Hydroxypropylmethylcellulose umfasst, bezogen auf das Gesamtgewicht dieser Zusammensetzung.

27. Verwendung nach einem der Ansprüche 18 bis 26, worin die pharmazeutische Zusammensetzung etwa 3 bis etwa 12 % des nicht ionischen hydrophilen Polymers umfasst, bezogen auf das Gesamtgewicht dieser Zusammensetzung.

28. Verwendung nach Anspruch 27, worin die pharmazeutische Zusammensetzung etwa 4 bis etwa 7 % des nicht ionischen hydrophilen Polymers umfasst, bezogen auf das Gesamtgewicht dieser Zusammensetzung.

29. Verwendung nach Anspruch 18, worin das Gewichtsverhältnis von Hydroxypropylmethylcellulose zu nicht ionischem hydrophilem Polymer von etwa 10:1 bis etwa 3:1 reicht.

30. Verwendung nach Anspruch 29, worin das Gewichtsverhältnis von Hydroxypropylmethylcellulose zu nicht ionischem hydrophilem Polymer von etwa 7:1 bis etwa 5:1 reicht.

31. Verwendung nach Anspruch 29, worin das Gewichtsverhältnis von Hydroxypropylmethylcellulose zu nicht ionischem hydrophilem Polymer 6:1 beträgt.

32. Pharmazeutische Zusammensetzung nach Anspruch 1, worin die HPMC einen Substitutionsgrad durch Hydroxypropyl (HP) von etwa 7 % bis 12 % hat.

33. Pharmazeutische Zusammensetzung nach Anspruch 1, worin die HPMC einen Substitutionsgrad durch Hydroxypropyl (HP) von etwa 7 % bis 9 % hat.

## Revendications

1. Composition pharmaceutique comprenant :
de la fluvastatine ou un de ses sels pharmaceutiquement acceptables ;
de l'hydroxypropyl méthyl cellulose ; et
un polymère hydrophile non-ionique sélectionné à partir du groupe composé d'hydroxyéthyl cellulose qui présente un poids moléculaire compris dans la gamme allant de 90 000 à 1 300 000, d'hydroxypropyl cellulose qui présente un poids moléculaire compris dans la gamme allant de 370 000 à 1 500 000, et de polyéthylène oxyde qui présente un poids moléculaire compris dans la gamme allant de 100 000 à 500 000.

2. Composition pharmaceutique selon la revendication 1, **caractérisé en ce que** le polymère hydrophile non-ionique est sélectionné à partir du groupe composé d'hydroxyéthyl cellulose qui présente un poids moléculaire compris dans la gamme allant de 1 000 000 à 1 300 000, d'hydroxypropyl cellulose qui présente un poids moléculaire compris dans la gamme allant de 850 000 à 1 500 000, et de polyéthylène oxyde qui présente un poids moléculaire compris dans la gamme allant de 150 000 à 300 000.

3. Composition pharmaceutique selon la revendication 2, **caractérisé en ce que** l'hydroxypropyl cellulose présente un poids moléculaire compris dans la gamme allant de 1 000 000 à 1 200 000.

4. Composition pharmaceutique selon la revendication 1, **caractérisé en ce que** le polymère hydrophile non-ionique est l'hydroxypropyl cellulose qui présente un poids moléculaire d'environ 1 150 000.

5. Composition pharmaceutique selon la revendication 2, **caractérisé en ce que** le polyéthylène oxyde présente un poids de 200 000.

6. Composition pharmaceutique selon la revendication 1 comprenant d'environ 5 à environ 50% en poids de fluvastatine, ou d'un de ses sels pharmaceutiquement acceptables, sur base du poids total de ladite composition.

7. Composition pharmaceutique selon la revendication 1 comprenant d'environ 20 à environ 40% en poids de fluvastatine, ou d'un de ses sels pharmaceutiquement acceptables, sur base du poids total de ladite composition.

8. Composition pharmaceutique selon la revendication 1 comprenant d'environ 15 à environ 50% en poids de l'hydroxypropyl méthyl cellulose, sur base du poids total de ladite composition.

9. Composition pharmaceutique selon la revendication 1 comprenant d'environ 20 à environ 40% en poids de l'hydroxypropyl méthyl cellulose, sur base du poids total de ladite composition.

10. Composition pharmaceutique selon la revendication 1 comprenant d'environ 3 à environ 12% en poids dudit polymère hydrophile non-ionique, sur base du poids total de ladite composition.

11. Composition pharmaceutique selon la revendication 1 comprenant d'environ 4 à environ 7 % en poids dudit polymère hydrophile non-ionique, sur base du poids total de ladite composition.

12. Composition pharmaceutique selon la revendication 1, **caractérisée en ce que** le rapport pondéral entre ladite hydroxypropyl méthyl cellulose et ledit polymère hydrophile non-ionique est compris dans la gamme allant d'environ 10:1 à environ 3:1.

13. Composition pharmaceutique selon la revendication 1, **caractérisée en ce que** le rapport pondéral entre ladite hydroxypropyl méthyl cellulose et ledit polymère hydrophile non-ionique est compris dans la gamme allant d'environ 7:1 à environ 5:1.

14. Composition pharmaceutique selon la revendication 1, **caractérisée en ce que** le rapport pondéral entre ladite hydroxypropyl méthyl cellulose et ledit polymère hydrophile non-ionique est de 6/1.

15. Composition pharmaceutique selon l'une quelconque des revendications précédentes comprenant :
84,24 mg de sodium de Fluvastavine,
8,42 mg de bicarbonate de potassium,
111,26 mg de Cellulose Microcristalline, NF, PH101, AVICEL®,
4,88 mg de Povidone,
16,25 mg de HPC, NF, KLUCEL® HXF,
97,50 mg de HPMC, METHOCEL® K 100LV,
2,44 mg de stéarate de magnésium, et
9,75 mg d'OPADRY ® jaune.

16. Composition pharmaceutique selon l'une des revendications 1 à 14 comprenant :
84,25 mg de sodium de Fluvastavine,
8,42 mg de bicarbonate de potassium,
111,2 mg de Cellulose Microcristalline, NF, PH101, AVICEL®,
4,88 mg de Povidone,
16,25 mg de HPC, NF, KLUCEL® HXF,
32,50 mg de HPMC, METHOCEL® K 100LV,
32,50 mg de HPMC, METHOCEL® K 15M,
32,50 mg de HPMC, METHOCEL® K 4M,
2,44 mg de stéarate de magnésium, NF, et
9,75 mg d'OPADRY ® jaune, YS-1-6347-G.

17. Composition pharmaceutique selon l'une des revendications 1 à 14 comprenant :
168,48 mg de sodium de Fluvastavine,
8,42 mg de bicarbonate de potassium,
65 mg de Cellulose Microcristalline, NF, PH101, AVICEL®,
20,5 mg de Povidone,
20,5 mg de HPC, NF, KLUCEL® HXF,
110,7 mg de HPMC, METHOCEL® K 100LV,
12,3 mg de HPMC, METHOCEL®,
4,1 mg de stéarate de magnésium, NF (1%), et
12,3 mg d'OPADRY ® rouge.

18. Utilisation d'un composition pharmaceutique comprenant :
de la fluvastatine ou un de ses sels pharmaceutiquement acceptables ;
de l'hydroxypropyl méthyl cellulose ; et
un polymère hydrophile non-ionique sélectionné à partir du groupe composé d'hydroxyéthyl cellulose qui présente un poids moléculaire compris dans la gamme allant de 90 000 à 1 300 000, d'hydroxypropyl cellulose qui présente un poids moléculaire compris dans la gamme allant de 100 000 à 500 000, et de polyéthylène oxyde qui présente un poids moléculaire compris dans la gamme allant de 100 000 à 500 000.

19. Utilisation selon la revendication 18, **caractérisée en ce que** le polymère hydrophile non-ionique est sélectionné à partir du groupe composé d'hydroxyéthyl cellulose qui présente un poids moléculaire compris dans la gamme allant de 1 000 000 à 1 300 000, d'hydroxypropyl cellulose qui présente un poids moléculaire compris dans la gamme allant de 850 000 à 1 500 000, et de polyéthylène oxyde qui présente un poids moléculaire compris dans la gamme allant de 150 000 à 300 000.

20. Utilisation selon la revendication 19, **caractérisée en ce que** l'hydroxypropyl cellulose présente un poids moléculaire compris dans la gamme allant de 1 000 000 à 1 200 000.

21. Utilisation selon la revendication 19, **caractérisée en ce que** le polymère hydrophile non-ionique présente un poids moléculaire d'environ 1 150 000.

22. Utilisation selon la revendication 19, **caractérisée en ce que** le polyéthylène oxyde présente un poids moléculaire d'environ 200 000.

23. Utilisation selon l'une quelconque des revendications 18 à 21, **caractérisée en ce que** la composition comprend, sur base du poids total de ladite composition, d'environ 5 à environ 50% de fluvastatine ou d'un de ses sels pharmaceutiquement acceptables.

24. Utilisation selon la revendication 23, **caractérisée en ce que** la composition comprend, sur base du poids total de ladite composition, d'environ 20 à environ 40 % de fluvastatine ou d'un de ses sels pharmaceutiquement acceptables.

25. Utilisation selon l'une quelconque des revendications 18 à 24, **caractérisée en ce que** la composition comprend, sur base du poids total de ladite composition, d'environ 15 à environ 50% de ladite hydroxypropyl méthyl cellulose.

26. Utilisation selon la revendication 25, **caractérisée en ce que** la composition comprend, sur base du poids total de ladite composition, d'environ 20 à environ 40 % de ladite hydroxypropyl méthyl cellulose.

27. Utilisation selon l'une quelconque des revendications 18 à 26, **caractérisée en ce que** la composition comprend, sur base du poids total de ladite composition, d'environ 3 à environ 12% dudit polymère hydrophile non-ionique.

28. Utilisation selon la revendication 27, **caractérisée en ce que** la composition comprend, sur base du poids total de ladite composition, d'environ 4 à environ 7% dudit polymère hydrophile non-ionique.

29. Utilisation selon la revendication 18, **caractérisée en ce que** le rapport pondéral entre ladite hydroxypropyl méthyl cellulose et ledit polymère hydrophile non-ionique est compris dans la gamme allant d'environ 10:1 à environ 3:1.

30. Utilisation selon la revendication 29, **caractérisée en ce que** le rapport pondéral entre ladite hydroxypropyl méthyl cellulose et ledit polymère hydrophile non-ionique est compris dans la gamme allant d'environ 7:1 à environ 5:1.

31. Utilisation selon la revendication 29, **caractérisée en ce que** le rapport pondéral entre ladite hydroxypropyl méthyl cellulose et ledit polymère hydrophile non-ionique est de 6/1.

32. Composition pharmaceutique selon la revendication 1, **caractérisée en ce que** HPMC possède un degrés de substitution de l'hydroxypropyle (HP) d'environ 7 à 12%.

33. Composition pharmaceutique selon la revendication 1, **caractérisée en ce que** HPMC possède un degrés de substitution de l'hydroxypropyle (HP) d'environ 7 à 9%.
